# EUROPEAN PATENT SPECIFICATION

(11) **EP 3 914 335 B1**
(45) Date of publication and mention of the grant of the patent: **05.03.2025**
(21) Application number: 20744407.6
(22) Date of filing: 24.01.2020
(51) Int. Cl.: A61N 1/04, A61N 1/18, A61N 1/36, A63B 24/00

(54) **ELECTRICAL MUSCLE STIMULATION DEVICES**
ELEKTRISCHE MUSKELSTIMULATIONSVORRICHTUNGEN
DISPOSITIFS DE STIMULATION MUSCULAIRE ÉLECTRIQUE

(30) Priority: 25.01.2019 US 201962797050 P
(43) Date of publication of application: 01.12.2021
(73) Proprietor: Therabody, Inc., Los Angeles, CA 90025 (US)
(72) Inventor: PISAREV, Alexey, Carlsbad, CA 92010 (US)
(74) Representative: Marks & Clerk LLP
(86) International application number: PCT/US2020/015080
(87) International publication number: WO 2020/154680

(56) References cited:
- EP-A1- 3 395 401
- WO-A1-2016/110804
- WO-A1-2017/163131
- WO-A2-2012/003451
- US-A1- 2016 213 924
- US-A1- 2016 346 543
- US-A1- 2017 056 643
- US-A1- 2018 200 514
- US-A1- 2018 304 074

## Description

### CROSS-REFERENCE TO RELATED APPLICATIONS

This application claims priority to U.S. Provisional Application No. 62/797,050 filed on January 25, 2019.

### FIELD

The present disclosure generally relates to electrical muscle stimulation, and, in particular, the present disclosure relates to devices, systems, and methods for an enterprise system for muscle stimulation, user feedback for muscle stimulation, and real time (or near real-time) adjustments for muscle stimulation.

### BACKGROUND

Electrical stimulation devices have a wide range of real world applications including uses in treatment, therapy, relaxation, fitness, athletic performance enhancement, entertainment, and the like. For example, electrodes may be attached to a user and the electrical stimulation device may transmit impulses to the user to stimulate and engage muscle(s) of the user, e.g., as during a workout. While offering a plurality of benefits, use of the electrical stimulation devices have been limited, for example, due to unwieldiness, difficulty of operation, and/or limited functionalities. There remains a need for improved muscle stimulation systems and techniques.
EP3395401 relates to an EXERCISE INSTRUMENT CONTROLLER AND EXERCISE INSTRUMENT CONTROL PROGRAM According to the abstract of this document there is provided an exercise instrument controller, a type determination unit that specifies, from among a plurality of types, the type of exercise for which an exercise instrument is used. An association determination unit specifies association between a body part that is exercised and the exercise instrument based on the type that has been specified. A setting processing unit sets an operation detail of the exercise instrument based on user's manipulation. An instrument control unit controls, by transmitting information indicating an operation detail for each exercise instrument based on the association between the body part and the exercise instrument that has been specified and the operation detail that has been set, exercise by the exercise instrument. A display control unit controls a screen display regarding the association between the body part and the exercise instrument, the operation detail, and an exercising status.

### SUMMARY

The devices disclosed herein generally relate to electrical muscle stimulation, and, in particular, to an enterprise system for muscle stimulation, user feedback for muscle stimulation, and real-time (or near real-time) adjustments for muscle stimulation.

In one aspect, a system for muscle stimulation may include a first computing device associated with a controlling user, and a first set of electrical muscle stimulation (EMS) devices engaged with a first end user of the system, where one or more EMS devices in the first set of EMS devices is in communication with a first processor and a first memory, and where one or more of the first processor and the first memory is in communication with the first computing device and is configured to receive and store instructions from the first computing device. The system may also include a second set of EMS devices engaged with a second end user of the system, where one or more EMS devices in the second set of EMS devices is in communication with a second processor and a second memory, and where one or more of the second processor and the second memory is in communication with the first computing device and is configured to receive and store instructions from the first computing device. The system may also include one or more communications channels between the first computing device and (i) one or more of the first processor and the first memory and (ii) one or more of the second processor and the second memory, the one or more communications channels facilitating operation of the first set of EMS devices and the second set of EMS devices via the first computing device, and the one or more communications channels switchable to stop communication from the first computing device after a predetermined transmission of a stimulation program for independent implementation of the stimulation program by an EMS device.

Implementations may include one or more of the following features. One or more communications channels may be configured to transmit data containing one or more stimulation programs from the first computing device to (i) one or more of the first processor and the first memory and (ii) one or more of the second processor and the second memory for implementation of the one or more stimulation programs on one or more of the EMS devices in the first set of EMS devices and one or more of the EMS devices in the second set of EMS devices. After transmission of the data, one or more communications channels may be configured to stop transmissions between the first computing device and one or more of the first processor, the first memory, the second processor, and the second memory. One or more of the first processor and the first memory may be integral with one or more of the EMS devices in the first set of EMS devices. One or more of the second processor and the second memory may be integral with one or more of the EMS devices in the second set of EMS devices. The system may further include a second computing device associated with the first end user of the system, where one or more of the first processor and the first memory is disposed on the second computing device. The second computing device may be separate and distinct from, but in communication with, the first set of EMS devices. The second computing device may be one or more of a smartphone and a tablet. The system may further include a third computing device associated with the second end user of the system, where one or more of the second processor and the second memory is disposed on the third computing device. The third computing device may be separate and distinct from, but in communication with, the second set of EMS devices. The third computing device may be one or more of a smartphone and a tablet. One or more of the first memory and the second memory may be configured to store the stimulation program. One or more of the first memory and the second memory may be configured to store historical data related to EMS devices and stimulation sessions. The controlling user may be one or more of a doctor and a physical therapist. The first end user and the second end user may be patients of the controlling user. The first computing device may be one or more of a smartphone and a tablet.

These and other features, aspects, and advantages of the present teachings will become better understood with reference to the following description, examples, and appended claims.

### BRIEF DESCRIPTION OF THE DRAWINGS

The foregoing and other objects, features and advantages of the devices, systems, and methods described herein will be apparent from the following description of particular embodiments thereof, as illustrated in the accompanying drawings. The drawings are not necessarily to scale, emphasis instead being placed upon illustrating the principles of the devices, systems, and methods described herein. In the drawings, like reference numerals generally identify corresponding elements.
Fig. 1 illustrates an enterprise system for muscle stimulation, in accordance with a representative embodiment.
Fig. 2 is a flow chart of a method for muscle stimulation using an enterprise system, in accordance with a representative embodiment.
Fig. 3 is a flow chart of a method for muscle stimulation with user feedback, in accordance with a representative embodiment.
Fig. 4 is a flow chart of a method for controlling a muscle stimulation session, in accordance with a representative embodiment.

### DETAILED DESCRIPTION

The embodiments will now be described more fully hereinafter with reference to the accompanying figures, in which preferred embodiments are shown. The foregoing may, however, be embodied in many different forms and should not be construed as limited to the illustrated embodiments set forth herein. Rather, these illustrated embodiments are provided so that this disclosure will convey the scope to those skilled in the art.

All documents mentioned herein are hereby incorporated by reference in their entirety. References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and so forth.

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately" or the like, when accompanying a numerical value, are to be construed as indicating a deviation as would be appreciated by one of ordinary skill in the art to operate satisfactorily for an intended purpose. Similarly, words of approximation such as "about," "approximately," or "substantially" when used in reference to physical characteristics, should be understood to contemplate a range of deviations that would be appreciated by one of ordinary skill in the art to operate satisfactorily for a corresponding use, function, purpose, or the like. Ranges of values and/or numeric values are provided herein as examples only, and do not constitute a limitation on the scope of the described embodiments. Where ranges of values are provided, they are also intended to include each value within the range as if set forth individually, unless expressly stated to the contrary. The use of any and all examples, or exemplary language ("e.g.," "such as," or the like) provided herein, is intended merely to better illuminate the embodiments and does not pose a limitation on the scope of the embodiments. No language in the specification should be construed as indicating any unclaimed element as essential to the practice of the embodiments.

In the following description, it is understood that terms such as "first," "second," "top," "bottom," "up," "down," and the like, are words of convenience and are not to be construed as limiting terms unless specifically stated to the contrary.

In general, the devices, systems, and methods disclosed herein generally relate to electrical muscle stimulation, and, in particular, to an enterprise system for muscle stimulation, user feedback for muscle stimulation, and real-time (or near real-time) adjustments for muscle stimulation. It will be understood that the present teachings may include, supplement, build-upon, replace, or improve any of the devices, systems, and methods disclosed in Int'l App. No. PCT/IB2017/000383 filed on March 22, 2017 (published as WO/2017/163131), which claims priority to U.S. Prov. App. No. 62/366,299 filed on July 25, 2016 and U.S. Prov. App. No. 62/311,811 filed on March 22, 2016,

Thus, the devices, systems, and methods disclosed herein may generally provide improved functionality and usability of electrical stimulation, or electrical muscular stimulation (EMS) devices. The EMS devices as described herein may be programmed, or otherwise structurally and programmatically configured to generate and/or transmit electrical impulses to a user. The EMS devices may thus include a device configured to implement a stimulation session (which may be referred to herein as a muscle stimulation session, a stimulation program, a session, and the like), e.g., on a user. A stimulation session may refer to a session with a beginning and an end during which electrical impulses are transmitted to the user via the EMS device for the purpose of treatment, therapy, relaxation, fitness, athletic performance enhancement, entertainment, combinations thereof, and the like. In some instances, the systems, methods, and devices of the present disclosure may help improve muscular fatigue resistance (e.g., endurance), increase muscular strength and power, improve muscular endurance and strength, improve muscle recovery (e.g., through increased blood flow), and/or potentiate muscle. In some instances, a stimulation session may refer to a session with a beginning and an end implemented via a set of instructions (e.g., non-transitory computer code, a computer program, and the like).

In general, the EMS devices described herein may be utilized for a variety of applications. For example, the EMS devices may be utilized for the prevention of muscle atrophy. In this manner, an EMS device may transmit electrical impulses to muscles so as to mimic neural impulses from the brain which may stimulate muscles and prevent atrophy for patients unable to utilize certain muscles. The EMS devices may also or instead be utilized for muscle relaxation or pain reduction. For example, electrical impulses transmitted via the EMS device may counteract neural impulses that cause muscle spasms which may aid in relaxation. The EMS devices may also or instead be utilized for entertainment or general stimulation. For example, the EMS devices may be utilized as a medium to deliver a stimulus in connection with modes of entertainment (e.g., virtual reality). The EMS devices may also or instead be utilized for medical purposes. For example, electrical impulses transmitted via the EMS devices may increase blood circulation, which may act to prevent blood clots and/or increase healing. Electrical impulses transmitted via the EMS devices may also or instead aid in rehabilitation of muscles, or may aid in the contraction of muscles as needed (e.g., drop foot assistance). The EMS devices may also or instead be utilized for general fitness or athletic performance enhancement. For example, electrical impulses transmitted via the EMS devices may assist in recovery from workouts or may supplement workouts by mimicking muscle activity.

An EMS device may include a main body, which may also be referred to herein as a central body, and may refer to a component of the EMS device used to generate electrical impulses. For example, the central body may generate an electrical impulse by accepting a current from a battery (e.g., onboard the EMS device) or from an electrical outlet or other power source. In some instances, an EMS device may further include one or more electrodes via which electrical impulses may be transmitted to a user. An EMS device may also or instead include a pulse generator. The pulse generator may be programmed, or otherwise configured to generate electrical impulses, e.g., in response to execution of a stimulation session. The pulse generator may be programmed, or otherwise configured to generate electrical impulses by accepting current from a battery or another power source such as an electrical outlet. In some instances, the EMS device may include a battery, which may for example include a replaceable battery or an integrated battery. The battery may also or instead include a rechargeable battery, such as a rechargeable lithium battery. In some instances, a port may be provided for recharging of the battery, such as a micro-USB port.

An EMS device may include stimulation channels, which can refer to an output channel for the generated electrical impulses. For example, an EMS device may include any number of stimulation channels. In some instances, the central body may comprise 1, 2, 3, 4, 5, 6, 7, 8, 9, 10 or more stimulation channels. The number of stimulation channels may correspond to a number of independent output channels for electrical impulses. For example, for each stimulation channel, a different electrical impulse may be generated and/or further transmitted by the EMS device. In some instances, an intensity level of all stimulation channels may be adjusted in accordance with a user's or program's instruction. Alternatively, an intensity level of each stimulation channel may be adjusted individually.

An EMS device may further include one or more electrodes, which may be integral with or coupled to pads or the like structurally configured for placement on a user's body, e.g., through the inclusion of an adhesive or the like. In some instances, the number of electrodes may correspond to a number of stimulation channels of the EMS device. The electrodes or pads may be configured to transmit electrical impulses generated by a pulse generator to a user, thereby stimulating the user. For example, electrical impulses generated at a central body may be transmitted to one or more pads via a wired connection.

In some instances, an EMS device, or a component in communication with the EMS device, may include a sensor system. The sensor system may be programmed or otherwise configured to sense signals such as signals from a muscle of a user, signals about a state of the sensor, and signals about an external environment. The sensor system may be utilized synergistically in conjunction with a stimulation session in order to provide useful information and to increase functionalities for an EMS device or session. In some instances, the integration of a sensing module onboard an EMS device (e.g., its main body) may ensure that the EMS device is simple to use and unobtrusive for a user while increasing possible applications for the EMS device. In some instances, the sensor system may be configured to record signals from a surface of a muscle, e.g., when it is contracted. The signals may include mechanical and/or electrical signals. For example, a mechanomyogram (MMG) or low frequency vibration may be observed and/or recorded utilizing the sensor system. The sensor system may include any suitable components for sensing the signals-for example, the sensor system may include accelerometers, gyroscopes, microphones, light emitters or detectors, and the like. For example, while a stimulation session is being implemented, muscles near the EMS device may undergo contraction due to transmitted electrical impulses, and the sensor system may sense signals (e.g., vibrations) from a surface of the muscles undergoing contraction and record these signals for analysis. The analysis may be performed on a processing unit of the EMS device; alternatively or in addition, sensed data may be transmitted to a remote device, and the analysis may be performed on that device or another device in direct or indirect communication therewith.

In some instances, an EMS device may include visual indicators, such as LEDs or the like. Such a visual indicator may be utilized to indicate different states of the EMS device. For example, the central body of an EMS device may include at least two LED indicators, where the first LED indicator may be used to indicate whether the device is turned on and/or whether the device is currently running, or executing a stimulation session, and the second LED indicator may be used to indicate a battery charging status.

An EMS device as described herein may be structurally configured to engage with a user's body to stimulate one or more muscles or muscle groups of a user. For example, an adhesive may enable an EMS device to be universally applied to any desired location on a user's body, e.g., on an arm, leg, knee, chest, abdomen, back, neck, shoulder, and so on. Other coupling of an EMS device with a user may also or instead be utilized without departing from the scope of this disclosure.

In some instances, a plurality of EMS devices may be in communication with one another. The plurality of the EMS devices my function together as a unit for implementing a stimulation session. Alternatively, or in addition, the plurality of EMS devices may implement a plurality of stimulation sessions (which may be the same or different). The plurality of EMS devices may implement stimulation sessions for a single person or a plurality of people, e.g., across different locations. In some instances, the plurality of EMS devices may be controlled by a computing device to implement stimulations that are varied and expansive in scope, while each EMS device remains simple to apply and unobtrusive. Data collected via the plurality of EMS devices may further be transmitted to a computing device for convenience of tracking and storage and may further be uploaded to a server, e.g., a cloud server or the like.

In some instances, a platform may be provided for use with the EMS devices. The platform may include a database or a server where stimulation programs may be managed (e.g., created, planned, scheduled, and so on). In some instances, data tracked by an EMS device and/or a computing device may be uploaded to the platform for ease of management and monitoring. The platform may act as a central database for managing and tracking stimulation sessions. In this manner, the platform may provide a convenient tool for managing and tracking stimulation sessions for the end users. For example, the platform may provide a convenient tool for specialized technicians, healthcare professionals, or other service providers to manage and track stimulation sessions for end users who may be clients or patients.

### Enterprise System for Muscle Stimulation

An enterprise system for muscle stimulation may feature the ability for a controlling user-e.g., a doctor, a trainer, or a physical therapist-to control multiple stimulation devices simultaneously, e.g., on different end users (e.g., patients).

Wireless communication standards such as Wi-Fi and Bluetooth may be limited by the number of communication channels that can be used for hopping within a certain radius without major interference between one another. Although frequency hopping is typically used, it is not always a guarantee for reliable communication. Also, on the other side, a single smartphone or tablet can usually only be simultaneously connected to a particularly limited number of devices (e.g., 6 to 8 devices) through short-range communication protocols, and these restrictions are generally set on the operating system/hardware drivers and a communication hardware chip being used.

With an enterprise system for muscle stimulation according to the present teachings, however, a controlling user may utilize a single computing device (e.g., a smartphone or tablet) or controller to initiate and control the flow and parameters of multiple stimulation programs on multiple end users engaged with multiple (wirelessly) connected muscle stimulators. In contrast, without the benefit of the present teachings, typical smartphones with typical Bluetooth restrictions may not allow for such a system because of these restrictions.

The enterprise system for muscle stimulation may include firmware for wirelessly connecting to and operating muscle stimulators, e.g., where the firmware is configured to allow a computing device (e.g., a smartphone) to wirelessly upload a complete stimulation program with all relevant parameters into a memory (e.g., a memory of a muscle stimulator or a device connected thereto), launch the stimulation program, and then disconnect wireless communication. In this manner, while wireless communication to a muscle stimulator is disconnected, the muscle stimulator may still execute the stimulation program while continuing to maintain stimulation parameters in an autonomous mode.

The enterprise system may include a mobile application or platform that allows users to select a relevant stimulation program, wirelessly connect to "free" muscle stimulators nearby, and allow users to upload and launch a muscle stimulation session on these muscle stimulators, where a stimulation session can be launched on a single stimulator or multiple stimulators. Then, if needed and depending on how many stimulation sessions have already started, the application can automatically disconnect from certain muscle stimulators and allow the users to either connect to the muscle stimulators that are already running a session (e.g., to make an adjustment to one or multiple stimulation parameters such as intensity, or to pause, resume, or stop a stimulation session) or to initiate a new session with other "free" muscle stimulators.

A mobile application may also or instead be configured to connect and to disconnect to certain muscle stimulators from time to time in the background in order to update to better control and reflect the flow of actively running stimulation sessions.

Thus, as described herein, because many wireless communication technologies (e.g., Bluetooth) have a limited number of channels (e.g., six channels that can be operated at one time), using the present teachings, devices can download or otherwise receive stimulation sessions from an application and then go off-line so that additional devices can be communicated with wirelessly. This can allow a user to control a potentially unlimited number of devices, e.g., by toggling back and forth between the devices, bringing them on-line and off-line as needed or desired. A user may also or instead monitor the progress of a stimulation program or session on each device through a mobile application or the like.

It will be understood that, although described in the context of electrical muscle stimulation devices, the present teachings could be used for other therapies and treatments. Similarly, although described in the context of Bluetooth, the present teachings could be used for another wireless technology or other communication protocols.

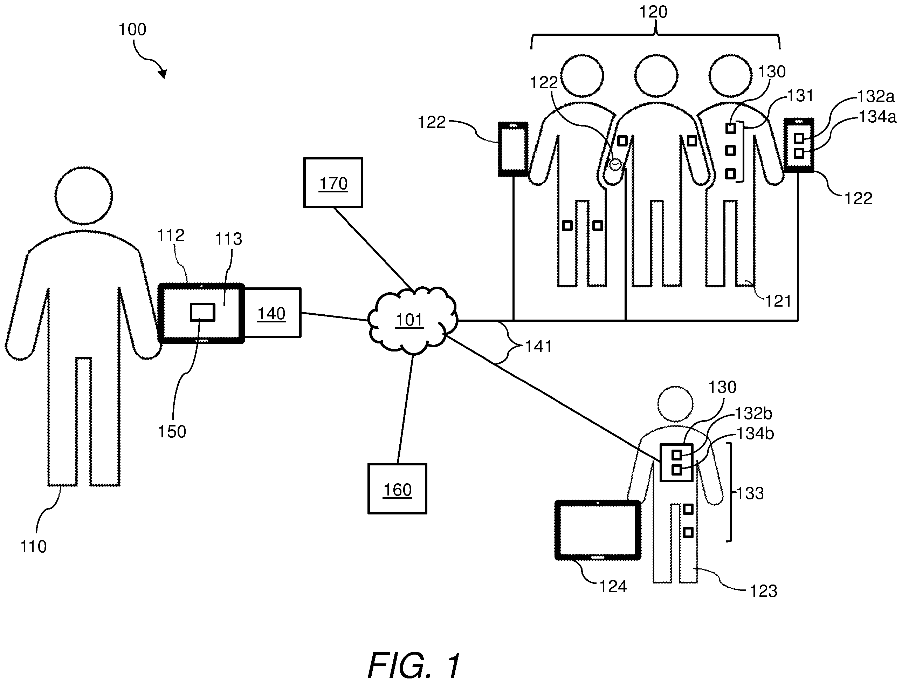

Fig. 1 illustrates an enterprise system for muscle stimulation, in accordance with a representative embodiment. The system 100 may include a controlling user 110 having a first computing device 112, a plurality of end users 120 having second computing devices 122, a plurality of electrical muscle stimulation (EMS) devices 130 having a processor and a memory (or otherwise in communication with a processor and a memory), a communications interface 140, a mobile application 150, and other resources 160 (e.g., other hardware or external resources), where one or more of the components of the system 100 are in communication through, or are otherwise connected over, a data network 101.

As stated above, the controlling user 110 may be one or more of a doctor, trainer, physical therapist, and the like, and the plurality of end users 120 may be patients, clients, or the like of the controlling user 110. In certain implementations, the controlling user 110 may also or instead be a user in which one or more EMS devices 130 are engaged-e.g., a user upon which a stimulation session can be implemented. That is, in certain implementations, the controlling user 110 may be controlling one or more EMS devices 130 engaged to themselves, in addition to or instead of controlling one or more EMS devices 130 engaged to other end users 120. The first computing device 112 may be associated with the controlling user 110-e.g., the first computing device 112 may be one or more of a smartphone and a tablet associated with the controlling user 110. In general, the system 100 may be configured such that a controlling user 110, using the first computing device 112, controls multiple EMS devices 130 simultaneously, and/or the system 100 may be configured such that a controlling user 110 uploads a complete stimulation program, launches the program, disconnects in favor of an autonomous mode, reconnects, and/or alters the stimulation program.

The plurality of EMS devices 130 may include any as described in Int'l App. No. PCT/IB2017/000383 filed on March 22, 2017 (published as WO/2017/163131). For example, one or more of the EMS devices 130 may be programmed to, or otherwise configured to, generate electrical impulses and transmit the electrical impulses to an end user 120, and, in some instances, an EMS device 130 may implement one or more stimulation sessions. An EMS device 130 may include a central body, one or more pads (e.g., where the pads include one or more electrodes), and/or other various components.

One or more of the EMS devices 130 may include, or may otherwise be in communication with, a processor and a memory. In certain implementations, the plurality of EMS devices 130 may be connected to at least two different end users 120. In this manner, the system 100 may include a first set of EMS devices 131 engaged with a first end user 121 of the system 100, and a second set of EMS devices 133 engaged with a second end user 123 of the system 100. One or more EMS devices 130 in the first set of EMS devices 131 may be in communication with a first processor 132a and a first memory 134a, where one or more of the first processor 132a and the first memory 134a is in communication with the first computing device 112 and is configured to receive and store instructions from the first computing device 112. Similarly, one or more EMS devices 130 in the second set of EMS devices 133 may be in communication with a second processor 132b and a second memory 134b, where one or more of the second processor 132b and the second memory 134b is in communication with the first computing device 112 and is configured to receive and store instructions from the first computing device 112. One or more of the processor and memory described herein may be disposed on an EMS device 130, and/or one or more of the processor and memory described herein may be disposed on a separate computing device. For example, one or more of the first processor 132a and the first memory 134a may be integral with one or more of the EMS devices 130 in the first set of EMS devices 131; similarly, one or more of the second processor 132b and the second memory 134b may be integral with one or more of the EMS devices 130 in the second set of EMS devices 133. Also, or instead, one or more of the first processor 132a and the first memory 134a may be disposed on the second computing device 122 associated with the first end user 121 of the system 100, and/or one or more of the second processor 132b and the second memory 134b may be disposed on a third computing device 124 associated with the second end user 123 of the system 100.

The first end user 121 and the second end user 123 may be located in the same geographic location or different geographic locations. Further, it will be understood that the first end user 121 and the second end user 123 may be the same individual in certain embodiments-e.g., where the first set of EMS devices 131 and the second set of EMS devices 133 are engaged with different body sections or muscle groups of the individual. It will be further understood that the EMS devices 130 included in the system 100 need not all be the same. For example, one or more EMS devices 130 in the first set of EMS devices 131 may be different from one or more EMS devices 130 in the second set of EMS devices 133. Also, or instead, an EMS device 130 in the first set of EMS devices 131 may be different from another EMS device 130 in the first set of EMS devices 131, and an EMS device 130 in the second set of EMS devices 133 may be different from another EMS device 130 in the second set of EMS devices 133. Where different EMS devices 130 are used in the system 100, the difference may be related to one or more of a shape or other structural difference, a difference in hardware or software (e.g., hardware or software integral therewith or otherwise in communication therewith), a difference in operating parameters or operating capability, and the like.

The system 100 may further include a controller 170, which may assist the controlling user 110 in operating the EMS devices 130. Thus, the EMS devices 130 may be in communication with a controller 170 that is configured to operate the EMS devices 130, e.g., including switching on/off communication channels 141 with the EMS devices 130 based on signals received from the EMS devices 130, or instructions received from the controlling user 110, a processor, or otherwise. In general, the controller 170 may be electrically coupled in a communicating relationship (e.g., an electronic communication) with any of the components of the system 100. In general, the controller 170 may be operable to control the components of the system 100, and may include any combination of software and/or processing circuitry suitable for controlling the various components of the system 100 described herein including without limitation processors, microprocessors, microcontrollers, application-specific integrated circuits, programmable gate arrays, and any other digital and/or analog components, as well as combinations of the foregoing, along with inputs and outputs for transceiving control signals, power signals, sensor signals, communication signals, and the like. In certain implementations, the controller 170 may include a processor or other processing circuitry with sufficient computational power to provide related functions such as executing an operating system or the mobile application 150, providing a graphical user interface, setting and providing rules and instructions for operation of a component of the system 100, converting sensed information into instructions, notifications, and the like, and operating a web server or otherwise hosting remote operators and/or activity through one or more communications interfaces 140. In certain implementations, the controller 170 may include a printed circuit board, an Arduino controller or similar, a Raspberry Pi controller or the like, a prototyping board, or other computer related components.

The controller 170, or another component in the system 100, may thus provide instructions for operation of one or more components of the system 100, such as one or more EMS devices 130. It will be understood that the set of instructions that may be transmitted to different EMS devices 130 may or may not be different from one another-e.g., the instructions may be transmitted at different times or simultaneously, and different instructions may refer to different stimulation sessions, which may be implemented via different EMS devices 130 at different times or simultaneously.

The controller 170 may be a local controller disposed on one or more of the computing devices, EMS devices 130, another component of the system 100, or a remote device otherwise in communication with the system 100 and its components. For example, one or more of the controller 170 and a user interface in communication with the controller 170 may be disposed on an external component (e.g., a computing device such as a smartphone) in communication with the system 100 over the data network 101. By way of further example, the controller 170 may be disposed on an EMS device 130. For example, a set of EMS devices may comprise a master device. In some instances, a set of EMS devices 130 may include a single master device, which may be programmed to, or otherwise configured to, broadcast or distribute commands to a plurality of other EMS devices 130, e.g., via a wireless communication channel, where the commands may relate to implementation and/or execution of a stimulation session. Alternatively or in addition, such a master device may be configured to receive data or signals from other devices.

The processor (e.g., one or more of the first processor 132a and the second processor 132b) may include an onboard processor for an EMS device 130 or another component of the system 100. The processor may also or instead be disposed on a separate computing device that is connected to the system 100 or one or more of its components through the data network 101, e.g., using the communications interface 140, which may include a Wi-Fi or Bluetooth transmitter and receiver. The processor may be any as described herein or otherwise known in the art. The processor may be included on the controller 170, or it may be separate from the controller 170, e.g., it may be included on a computing device in communication with the controller 170 or another component of the system 100. In an implementation, the processor is included on, or is in communication with, a server that hosts the mobile application 150 for operating and controlling the system 100.

The memory (e.g., one or more of the first memory 134a and the second memory 134b) may be any as described herein or otherwise known in the art. The memory may contain computer code and may store data such as sequences of operation for one or more of the components of the system 100 (e.g., the EMS devices 130), sequences or content for notifications and alerts, historical data (e.g., previous inputs, measurements or sensed information, stimulation sessions or parameters thereof, and calculations), and so on. The memory may also or instead contain computer executable code stored thereon that provides instructions for the processor (e.g., one or more of the first processor 132a and the second processor 132b) for implementation. The memory may include a non-transitory computer readable medium. Thus, in certain implementations, one or more of the first memory 132a and the second memory 132b is configured to store the stimulation program. Also, or instead, one or more of the first memory 132a and the second memory 132b may be configured to store historical data related to EMS devices 130 and stimulation sessions-e.g., settings or parameters related thereto, sensor data related thereto, user options or feedback related thereto, scheduling and timing information, physiological data related to a user in response to EMS devices 130 and stimulation sessions, and so forth.

The system 100 may further include a computing device in communication with one or more of the components of the system 100-e.g., one or more of the first computing device 112, the second computing device 122, and the third computing device 124, where more or less computing devices are possible. In the context of the example implementation of the system 100 in Fig. 1, the system 100 may include a second computing device 122 associated with the first end user 121 of the system 100, and a third computing device 124 associated with the second end user 123 of the system 100. As discussed above, the processor and memory described herein may be disposed on one or more of the second computing device 122 and the third computing device 124. Furthermore, one or more of the second computing device 122 and the third computing device 124 may be separate and distinct from, but in communication with, the first set of EMS devices 131 and the second set of EMS devices 133, respectively. One or more of the second computing device 122 and the third computing device 124 may be a smartphone, a tablet, or the like.

The computing device-e.g., one or more of the first computing device 112, the second computing device 122, and the third computing device 124-may include any devices within the system 100 operated by operators or otherwise to manage, monitor, communicate with, or otherwise interact with other participants in the system 100. This may include desktop computers, laptop computers, network computers, tablets, smartphones, smart watches or other wearable computing devices, PDAs, or any other device that can participate in the system 100 as contemplated herein. In an implementation, the computing device is integral with another participant in the system 100-e.g., the computing device may be integral with an EMS device 130.

The computing device may include a user interface 113, e.g., in communication with a component of the system 100. Thus, a computing device may generally provide a user interface 113 for a mobile application 150, which may include a graphical user interface, a text or command line interface, a voice-controlled interface, and/or a gesture-based interface. In general, the user interface 113 may create a suitable display on a computing device for operator interaction. In implementations, the user interface 113 may control operation of one or more of the components of the system 100, as well as provide access to and communication with the controller 170, processor, and other resources 160. The user interface 113 may be maintained by a locally executing application on the computing device (e.g., the mobile application 150) that receives data from one or more of the components of the system 100. In other embodiments, the user interface 113 may be remotely served and presented on a computing device, such as where a web server provides information through one or more web pages or the like that can be displayed within a web browser or similar executing on the computing device. In implementations, the user interface 113 may also or instead be provided by and/or disposed on another participant in the system 100.

A computing device as described herein may generally be utilized for managing and tracking stimulation sessions. For example, a user may utilize a computing device, or an application on the computing device, to devise (e.g., create), select, schedule, or plan stimulation sessions and control parameters of stimulation sessions.

The data network 101 may be any network(s) or internetwork(s) suitable for communicating data and control information among participants in the system 100. This may include public networks such as the Internet, private networks, telecommunications networks such as the Public Switched Telephone Network or cellular networks using third generation (e.g., 3G or IMT-2000), fourth generation (e.g., LTE (E-UTRA) or WiMAX-Advanced (IEEE 802.16m) and/or other technologies, as well as any of a variety of corporate area or local area networks and other switches, routers, hubs, gateways, and the like that might be used to carry data among participants in the system 100. The data network 101 may include wired or wireless networks, or any combination thereof, e.g., a Bluetooth or other similar communications protocol network. One skilled in the art will also recognize that the participants shown the system 100 need not be connected by a data network 101, and thus can be configured to work in conjunction with other participants independent of the data network 101. Thus, in some instances, one or more computing devices may be in direct or indirect communication with one or more EMS devices 130. For example, a computing device may communicate with one or more EMS devices 130 via wireless communication by utilizing a radio-frequency (RF) protocol or other wireless communication, e.g., by utilizing an ANT+, Bluetooth, Gazell protocol, or the like. In some instances, a computing device may transmit instructions for implementing a stimulation session on an EMS device 130, where data (e.g., data regarding parameters of a stimulation session or data from a sensor system) is recorded or tracked by the EMS device 130 or a computing device in communication therewith, and where this data may also be transmitted back to the computing device providing instructions to the EMS device 130.

Communication over the data network 101, or other communication between components of the devices or systems described herein, may be provided via one or more communications interfaces 140. The communications interface 140 may include, e.g., a Wi-Fi receiver and transmitter to allow the logic calculations to be performed on a separate computing device. This may include connections to smartphone applications and the like. More generally, the communications interface 140 may be suited such that any of the components of the system 100 can communicate with one another. Thus, the communications interface 140 may be present on one or more of the components of the system 100, although only shown coupled with the first computing device 112 for convenience in the figure. The communications interface 140 may include, or be connected in a communicating relationship with, a network interface or the like. The communications interface 140 may include any combination of hardware and software suitable for coupling the components of the system 100 to a remote device (e.g., a computing device such as a remote computer or the like) in a communicating relationship through a data network 101. By way of example and not limitation, this may include electronics for a wired or wireless Ethernet connection operating according to the IEEE 802.11 standard (or any variation thereof), or any other short or long range wireless networking components or the like. This may include hardware for short range data communications such as Bluetooth or an infrared transceiver, which may be used to couple into a local area network or the like that is in turn coupled to a data network such as the internet. This may also or instead include hardware/software for a WiMAX connection or a cellular network connection (using, e.g., CDMA, GSM, LTE, or any other suitable protocol or combination of protocols). Additionally, the controller 170 may be configured to control participation by the components of the system 100 in any network to which the communications interface 140 is connected, such as by autonomously connecting to the data network 101 to retrieve status updates and the like.

The system 100 may include other resources 160. In certain implementations, the other resources 160 may include sensors, cameras, power sources, switchboards, and the like. The other resources 160 may also or instead include input devices such as a keyboard, a touchpad, a computer mouse, a switch, a dial, a button, and the like, as well as output devices such as a display, a speaker or other audio transducer, light-emitting diodes or other lighting or display components, and the like. Other resources 160 of the system 100 may also or instead include a variety of cable connections and/or hardware adapters for connecting to, e.g., external computers, external hardware, external instrumentation or data acquisition systems, and the like.

The other resources 160 may also or instead include a server, a database or other data storage, a remote resource, a network interface, processing circuitry, and the like. Thus, other resources 160 such as other hardware or other software may be included in addition to, or instead of, components described above.

The system 100 may include one or more communications channels 141 between the first computing device 112 and (i) one or more of the first processor 132a and the first memory 134a and (ii) one or more of the second processor 132b and the second memory 134b. Stated otherwise, the first computing device 112 may be in communication with another computing device (or simply a processor and/or a memory) that is in communication with a plurality of EMS devices 130 (e.g., one or more of the first set of EMS devices 131 and the second set of EMS devices 133) over one or more communications channels 141, e.g., for wireless control of the EMS devices 130. In some implementations where an EMS device 130 includes an integral processor, memory, and/or communications interface 140, the first computing device 112 may be directly in communication with the EMS device 130 over one or more communications channels 141. Thus, the one or more communications channels 141 may facilitate operation of the first set of EMS devices 131 and the second set of EMS devices 133 via the first computing device 112.

Further, the one or more communications channels 141 may be switchable to stop communication between the first computing device 112 and an EMS device 130-or to otherwise cutoff communication or the transfer of data between the first computing device 112 and an EMS device 130-after a predetermined transmission of a stimulation program for independent implementation of the stimulation program by an EMS device 130.

The one or more communications channels 141 may be configured to transmit data containing one or more stimulation programs from the first computing device 112 to an EMS device 130. This may occur through a transmission of data (e.g., containing one or more stimulation programs) from the first computing device 112 to (i) one or more of the first processor 132a and the first memory 134a and (ii) one or more of the second processor 132b and the second memory 134b for implementation of a stimulation program on one or more of the EMS devices 130 in the first set of EMS devices 131 and one or more of the EMS devices 130 in the second set of EMS devices 133. After transmission of the data, the one or more communications channels 141 may be configured to stop transmissions between the first computing device 112 and one or more of the first processor 132a, the first memory 134a, the second processor 132b, and the second memory 134b.

A method according to the present teachings may include connecting to one or more first EMS devices, uploading or activating a first stimulation program to the first EMS devices, and disconnecting from the first EMS devices. The method may further include connecting to one or more second EMS devices (separate and distinct from the first EMS devices), uploading or activating a second stimulation program to the second EMS devices, and disconnecting from the second EMS devices. The method may further include toggling communication and control between the first and second EMS devices. In other words, a method of controlling a plurality of muscle stimulation sessions according to the present teachings may include: wirelessly connecting to one or more first EMS devices; activating a first stimulation program on the one or more first EMS devices; disconnecting from communication with the one or more first EMS devices; wirelessly connecting to one or more second EMS devices, the second EMS devices separate and distinct from the one or more first EMS devices; activating a second stimulation program on the one or more second EMS devices; disconnecting from communication with the one or more second EMS devices; and toggling communication and control between the one or more first EMS devices and the one or more second EMS devices.

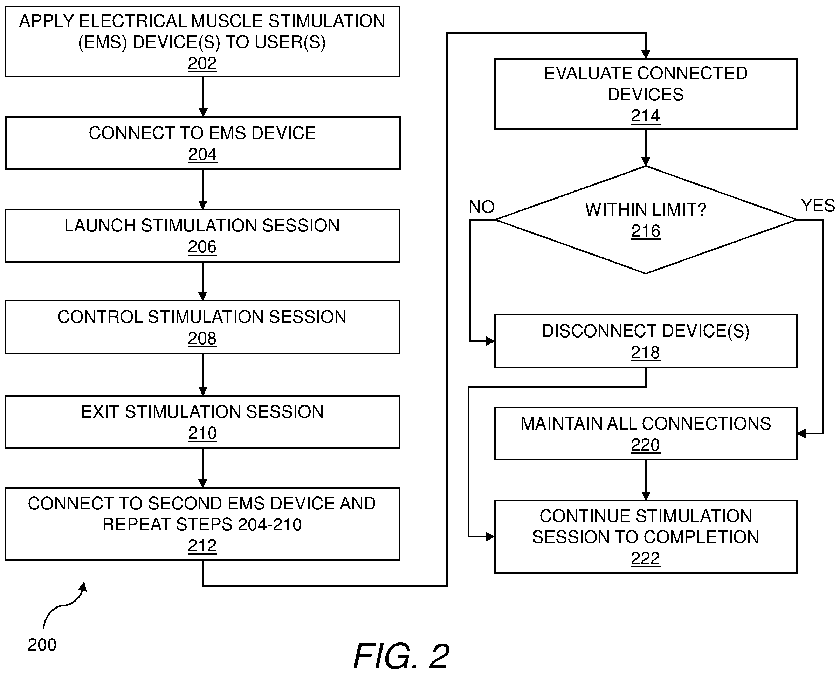

Fig. 2 is a flow chart of a method 200 for muscle stimulation using an enterprise system, in accordance with a representative embodiment. It will be understood that the method 200 may be performed, e.g., using the system 100 shown and described above with reference to Fig. 1. The method 200 may be used for controlling a plurality of muscle stimulation sessions on a plurality of EMS devices. It will be understood that the method 200 may be used for a single user controlling muscle stimulation sessions on his or herself, or for one or more users controlling muscle stimulation sessions experienced by one or more other, different users.

As shown in step 202, the method 200 may include applying one or more EMS devices to one or more users. For example, this may include having EMS devices applied to multiple patients (e.g., applying EMS devices to themselves, or having a medical professional or the like apply the EMS devices) for use in a stimulation session, where control of the stimulation session is performed by a single controlling user, such as a doctor or a physical therapist.

As shown in step 204, the method 200 may include connecting to an EMS device for control thereof. The connection may be established using a mobile application or the like, e.g., using one or more of the communication protocols described herein or known in the art (e.g., Bluetooth), and where the mobile application is accessible via the computing device of a controlling user. Thus, the method 200 may include wirelessly connecting to one or more first EMS devices, e.g., where the connection is established via a short-range communication protocol or the like. The method 200 may also or instead include establishing a connection between a computing device of a controlling user and at least a first EMS device for control thereof.

As shown in step 206, the method 200 may include launching a stimulation session. More specifically, the method 200 may include launching a stimulation session on a first EMS device via the computing device of a controlling user. In this manner, and by way of example, steps 202-206 may collectively entail a doctor selecting a patient from a patient list (e.g., using a mobile application or other similar software), attaching or connecting to one or more pre-registered EMS devices from the application, activating the EMS device(s) (e.g., the application may automatically recognize recently activated devices and connect to them), and launching a stimulation session for the selected patient or devices. Stated otherwise, the method 200 may further include selecting a user from a list of users for establishing the connection and launching the stimulation session on an EMS device associated with the selected user. Regardless, the method 200 may include activating a first stimulation program on one or more first EMS devices that were connected in step 204.

As shown in step 208, the method 200 may include controlling the stimulation session. More specifically, the method 200 may include controlling the stimulation session on at least a first EMS device via the computing device. Controlling the stimulation session may include one or more of starting the stimulation session, stopping the stimulation session, pausing the stimulation session, increasing a frequency of activation of an EMS device during the stimulation session, decreasing the frequency of activation of an EMS device during the stimulation session, increasing an intensity of an EMS device during the stimulation session, decreasing the intensity of an EMS device during the stimulation session, changing to a different stimulation session, and the like. Controlling the stimulation session may also or instead include adjusting a parameter thereof, such as a pulse width, a frequency of pulses, a length or frequency of a contraction phase, a length or frequency of a rest phase, a rest period, a ramp-up time, a ramp-down time, duty cycle parameters, a number of contractions, a duration, burst pulse parameters, waveform shape, interphase intervals, a stimulation length, a stimulation intensity, a stimulation session number, and the like.

As shown in step 210, the method 200 may include exiting the stimulation session, and as shown in step 212, the method 200 may include connecting to a second, different EMS device (e.g., on another user) and repeating one or more of steps 204-210. In this context, exiting the stimulation session may include exiting access to the stimulation session by the computing device of the controlling user, which can include disconnecting from an EMS device and/or otherwise stopping control of the EMS device. In other words, the method 200 may include disconnecting from communication with one or more first EMS devices that were connected in step 204, and wirelessly connecting to one or more second EMS devices, where the second EMS devices are separate and distinct from the first EMS devices. The method 200 may then include activating a second stimulation program (e.g., which can be the same as, different from, associated with, or unassociated with a first stimulation program that is/was operating on first EMS devices) on one or more of the second EMS devices. For example, the first stimulation program may be associated with the second stimulation program by the stimulation programs working in a coordinated or cooperating manner, which can be especially useful when the stimulation programs are being operated on a single user.

By way of example, a doctor may exit a stimulation session and repeat steps 204-210 for another patient or patients, e.g., where every patient receives his/her own pre-scheduled or ad hoc customized stimulation session. It will be understood that a stimulation session may continue for a patient even when the controlling user exits from the session. In this manner, connecting to a stimulation session may be used for monitoring or control thereof, but the stimulation session may otherwise carry on when a controlling user disconnects from communication with the EMS device running the session. Thus, the method 200 may include exiting access to one or more stimulation sessions via the computing device, wherein, after exiting access, a stimulation session continues to run on an EMS device. When connected or disconnected, the method 200 may include monitoring a stimulation session via the computing device.

It will be understood that, in the examples of the method 200 that include first and second EMS devices, the first EMS devices and the second EMS devices may be disposed on different users, or they may be disposed on the same user (e.g., where the first EMS devices and the second EMS devices are stimulating different muscle groups or body regions).

As shown in step 214, the method 200 may include evaluating the connected EMS devices. More specifically, the method 200 may include evaluating a plurality of EMS devices that are connected to the computing device of the controlling user for compliance with a rule, and disconnecting one or more of the plurality of EMS devices when there is noncompliance with the rule.

For example, as shown in step 216, the rule may set forth a predetermined limit for EMS devices connected with the computing device (e.g., where the predetermined limit is six EMS devices). In this manner, when the number of EMS devices exceeds a certain predetermined limit, the method 200 may proceed to step 218, whereas, when the number of EMS devices is within a certain predetermined limit, the method 200 may proceed to step 220. By way of example, when a doctor initiates a new stimulation session with a new set of EMS devices, an application or the like may determine how many EMS devices are already connected, and, if the total number of EMS devices exceeds a certain limit (e.g., six), the application may disconnect from one or more of the previously connected EMS devices and connect to the new EMS devices instead. In this manner, limits of certain communications protocols may be adhered to, while continuing to run stimulation sessions. That is, in certain implementations, disconnected devices may continue executing stimulation sessions in the background, even when disconnected from communication with a controlling user.

Other rules for connecting and disconnecting EMS devices (e.g., a rule unrelated to a limit on the number of EMS devices that are connected) are also or instead possible. For example, there may be a rule that prioritizes certain EMS devices over other EMS devices. In this manner, EMS devices may be disconnected based on priority. By way of further example, there may be a rule that sets forth disconnecting EMS devices based on timing-e.g., EMS devices may be disconnected based on priority and/or based on the progress of one or more stimulation sessions implemented on EMS devices.

Thus, as shown in step 218, the method 200 may include disconnecting an EMS device, e.g., when the number would otherwise exceed a certain limit. And, as shown in step 220, the method 200 may include maintaining connections to certain or all EMS devices, e.g., when the number does not exceed a certain limit. Thus, the method 200 may include disconnecting from communication with one or more of the first or second EMS devices, and toggling communication and control between the first EMS devices and the second EMS devices, e.g., depending upon a number of EMS devices that are able to be connected simultaneously. For example, when a total number of first EMS devices and second EMS devices is greater than 'n' devices, toggling may be useful because there may a maximum of 'n' devices that are able to be connected simultaneously (e.g., where 'n' equals six).

In certain implementations, the method 200 includes launching a second stimulation session on a second EMS device via the computing device (in addition to, or instead of, the first stimulation session that was previously implemented on a first EMS device), and controlling the second stimulation session on the second EMS device via the computing device (in addition to, or instead of, controlling the first stimulation session on the first EMS device). And, after establishment and/or controlling of the second stimulation session, the method 200 may include exiting access to the second stimulation session via the computing device. In certain implementations, after exiting access, the second stimulation session continues to run on at least the second EMS device.

As shown in step 222, the method 200 may include continuing stimulation sessions to completion, e.g., whether connected to the controlling user or otherwise. This may be accomplished through the stimulation programs being uploaded to a memory in communication with, or integral to, one or more EMS devices. That is, in certain implementations, the method 200 may include uploading the first stimulation program to a first memory associated with one or more of the first EMS devices, and uploading the second stimulation program to a second memory associated with one or more of the second EMS devices.

Thus, by way of example, using the method 200, when a doctor or the like reselects one of the older sessions that was previously disconnected (e.g., EMS devices used in that session were disconnected from communication), the application may automatically disconnect the same number of EMS devices used in other sessions and reconnect to the EMS devices for the currently selected session. In this manner, the method 200 may provide a doctor or other controlling user with full control of a stimulation session including the ability to dynamically change a stimulation program and control the flow of a stimulation session.

### User Feedback for Muscle Stimulation

Information obtained from a third-party system/application (e.g., Strava, Fitbit, and the like) may not be sufficient to create a specific stimulation program for a user. Often times, additional information may be needed or desired in order to create a fully customized session suitable for a user. To this end, the present teachings may include querying a user for additional information, and using that information in combination with third-party information to create a customized stimulation session.

Specifically, a technique may involve receiving information from one or more third-party systems (or from EMS devices directly, or from other sensors), and querying a user, e.g., with questions or the like to understand more about the user or an activity the user has undertaken. This may provide a more accurate classification of an activity reported back by third-party systems. For example, the technique may use tags, free text, and the like, e.g., to select muscle groups or body areas that might be feeling tired or painful, to estimate a degree of soreness or pain, to determine the availability of the user to run recovery or performance muscle stimulation sessions, and so on. This combined information may be used to generate or schedule one or more muscle stimulation sessions for the user, where these stimulation sessions can be uniquely customized for the user.

By way of example, using these techniques, a custom recovery program can be created. By way of further example, the recovery program can include a particular number of sub-phases that will fit into a discrete amount of time that a user currently has to devote to a muscle stimulation session, while still being long and substantial enough to flush out extra lactate from the user's blood, e.g., based on information received from a third-party system related to the time that the user has spent in physical activity over an estimated lactate threshold level. In this manner, sessions can be generated on-demand for the most fatigued/sore muscle groups, and follow-up recovery as well as lactate threshold training sessions can be developed for less fatigued muscle groups for subsequent execution, e.g., within 12 hours.

Upon completion of a stimulation session, techniques may also or instead include querying a user to provide feedback, e.g., in the form of multiple choice questions or in free text format (e.g., asking the user if they feel less pain or muscle soreness after a session compared to during or before, whether the user wants to run a session for a longer period of time, or whether the user would like to try different settings-e.g., have a stimulation session be more or less aggressive/intense). Feedback answers may thus be used to modify stimulation parameters of future customized stimulation sessions for the user. Feedback may also or instead include sensed feedback received during a stimulation session.

Over time, machine learning techniques and algorithms may analyze user responses, e.g., for similarity patterns to reduce the number of questions being asked or to provide a user with more intuitive or efficient controls (e.g., by pre-selecting the most-probable answers and/or only asking a user to confirm).

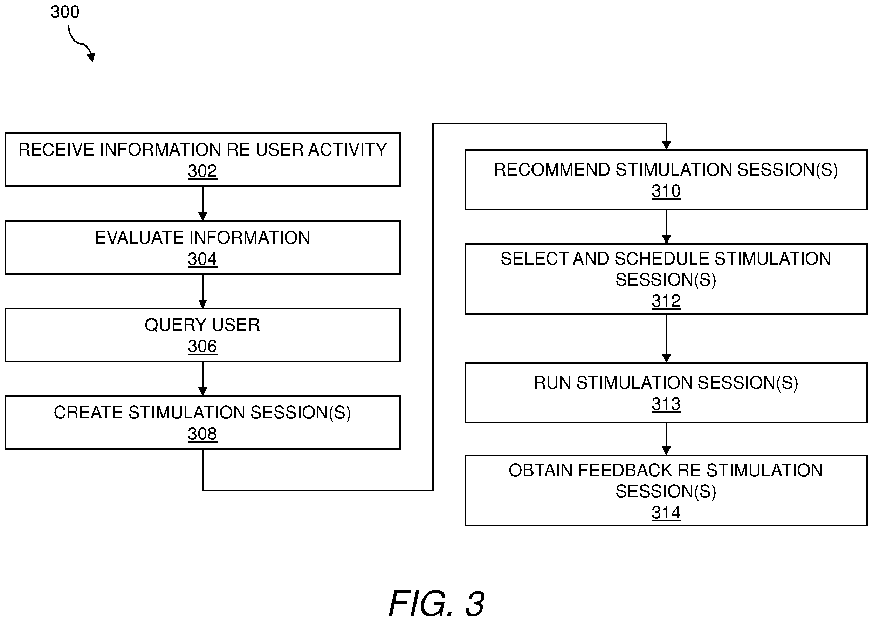

Fig. 3 is a flow chart of a method 300 for muscle stimulation with user feedback, in accordance with a representative embodiment. In general, the method 300 may include customizing or thoughtfully (and strategically) selecting muscle stimulation sessions, which may include querying a user for information and using that information in combination with third-party information to either create a customized stimulation session or select an existing stimulation session, or combinations thereof.

As shown in step 302, the method 300 may include receiving information regarding a user activity-e.g., receiving third-party information regarding a user activity of a user, where the third-party information includes data received from a third-party data source. In certain implementations, third-party information is received from the third-party data source in response to a query from a stimulation session application. For example, a server running a stimulation session application may receive information from a third-party system (such as Strava^{®} or the like) about recent activity (e.g., a running workout), where the server then sends any relevant push notifications or the like to a user through a mobile application or the like. In this manner, the mobile application or the like may send a push notification to the user with an invitation to schedule and run an EMS recovery session (or other stimulation session). Thus, more generally, the method 300 may include sending a notification to a user in response to receiving one or more of the third-party information and the user-based information. The notification may include an invitation to initiate or schedule a muscle stimulation session (see also step 312 described below).

Third-party information may include one or more of a workout type, a workout intensity, a workout length, calories burned, a measurement of physical activity (e.g., steps), weights or resistance used, injury information, and the like. Third-party information may also or instead include one or more of heart rate data, accelerometer data, breathing data, mechanomyography (MMG) readings, other physiological data, geographic or mapped or location data, environmental data (e.g., weather, temperature, humidity, etc.), and the like. In some instances, third-party information may be obtained from health-related or fitness-related applications. For example, health- and fitness-related application programs are commonly used to log or track information on a user (e.g., a number of steps taken). Non-limiting examples of application programs include Apple Health, Fitbit, Google Fit, JawBone Up, MapMyFitness, Mind Body, Moves, Nike+, RunKeeper, Strava, Under Armour Connected Fit, Wahoo Fitness, Withings, and Wodify. Communication to receive third-party information may be achieved using one or more application programming interfaces (APIs). An API may generally refer to a set of routines, protocols, or tools for building software applications that may interact with a given application. In any of the embodiments disclosed herein, communication between at least one application program and the API may include an API call.

As shown in step 304, the method 300 may include evaluating the information that is received, e.g., to determine or select an appropriate stimulation or recovery session for a particular user, or whether a customized stimulation or recovery session should be created.

As part of the information gathering and evaluation, as shown in step 306, the method 300 may include querying the user for additional, user-based information, and receiving the user-based information for use in creating or selecting a stimulation session for the user. Examples of such user-based information may include one or more of a workout type, a workout intensity, a workout length, and the like. User-based information may also or instead include one or more of an identification of one or more muscles for targeting in a muscle stimulation session, data related to muscle fatigue or muscle soreness, a user history of one or more of muscle stimulation sessions and user activities, physiological data, demographic data, health information (e.g., an electronic health record) or medical history, medication information, weight, body mass index (BMI), other user characteristics, and the like. Thus, one or more of the third-party information and the user-based information may include one or more of physical data, geographic data, measured data, and sensed data.

For example, a user may open a mobile application, where the application asks the user to provide classification of his/her activity, e.g., "Medium Intensity Running Session," or the user may be prompted to select from a list of activities. Similarly, the application may query the user to provide classification or identification for a desired stimulation session, e.g., using previously run sessions or activities as a guidepost. A user may also or instead be asked to specify muscle groups, which he/she believes require recovery, and/or to provide a level of muscle fatigue or muscle soreness for a muscle group. Such selected muscle fatigue/soreness levels may be saved in memory and may be automatically preloaded when a user selects a previously classified activity or stimulation session. A user may also or instead be asked to specify how much time they have available for a recovery session, e.g., less than 15 minutes, 15-20 minutes, 20-30 minutes, more than 30 minutes, and so on. Regarding any of the aforementioned information, or other information, the user may be queried by prompting the user to select from a list or the like. By way of example, querying the user may include prompting the user to select a workout type from a list of activities, where the user-based information includes the workout type. By way of further example, querying the user may also or instead include prompting the user to select one or more preferences related to muscle stimulation sessions, where the user-based information includes these preferences related to muscle stimulation sessions. One or more preferences related to muscle stimulation sessions provided by a user may include at least one of a duration, an intensity, a muscle group, an attribute related to one or more EMS devices, combinations thereof, and the like.

Evaluation of received information may include analyzing physiological data, physical data, geographic data, measured or sensed data, and so on. More generally, the method 300 may further include estimating a time the user spent above a predetermined lactate threshold level based on one or more of the third-party information and the user-based information. For example, heart rate or speed data from a third-party system may be used to estimate how much time a user spent exercising above a lactate threshold level. By way of further example, MMG readings may be analyzed to detect muscle performance parameters, e.g., a level of muscle fatigue. Also or instead, the analyzing step may relate to a gait, hand grip analysis of a user, and the like.

Thus, turning back to step 304, the method 300 may include evaluating the third-party information and the user-based information to select between a predetermined muscle stimulation session and a customized muscle stimulation session. In some instances, the aforementioned selection is not mutually exclusive. For example, a predetermined muscle stimulation session may be selected and then altered to create a customized muscle stimulation session. In other implementations, a predetermined muscle stimulation session may be selected and used without any alterations. Similarly, in certain implementations, a customized muscle stimulation session may be created from scratch.

As shown in step 308, the method 300 may include creating a customized stimulation session, or selecting a pre-established stimulation session, e.g., in response to the received and evaluated data. Alternatively, or additionally, as shown in step 310, the method 300 may include recommending one or more stimulation sessions, e.g., custom or otherwise, which may similarly be in response to the received and evaluated data. For example, depending on the information received (e.g., including an estimated or known amount of time spent above a lactate threshold level), a customized recovery session may be automatically generated by the application, e.g., focusing on the most fatigued/sore muscle groups and providing enough blood circulation and endorphin generation stimulation modalities to flush lactic acid from the blood of the user.

Thus, in certain implementations, when the predetermined muscle stimulation session is selected, the method 300 may include providing a recommendation to the user of one or more predetermined muscle stimulation sessions targeting one or more muscle groups. These predetermined muscle stimulation sessions may be selected to provide blood circulation and endorphin generation stimulation modalities for flushing lactic acid from blood of the user in the one or more targeted muscle groups. Further, in certain implementations, when the customized muscle stimulation session is selected, the method 300 may include creating one or more customized muscle stimulation sessions based on the third-party information and the user-based information. These customized muscle stimulation sessions may be created to provide blood circulation and endorphin generation stimulation modalities for flushing lactic acid from blood of the user in the one or more targeted muscle groups.

As shown in step 312, the method 300 may include selecting and scheduling a stimulation or recovery session. In this manner, the method 300 may include scheduling one or more of the predetermined muscle stimulation session and the customized muscle stimulation session. This may be manually done by the user or automatically by the application or the like. In certain aspects, if available user time is limited, then wider stimulation pulses may be used for a recovery program, and, at the same time, additional recovery sessions may be generated for the user for subsequent implementation. That is, the method 300 may further include scheduling one or more muscle stimulation sessions for implementation after one or more of the predetermined muscle stimulation session and the customized muscle stimulation session.

As shown in step 313, the method 300 may include running one or more of the predetermined muscle stimulation session and the customized muscle stimulation session on one or more EMS devices engaged with the user.

As shown in step 314, the method 300 may include obtaining feedback regarding a stimulation or recovery session. This may stem from subjective information provided by the user through queries or the like, objectively through measured or sensed information, or a combination thereof. Thus, the method 300 may include querying the user for feedback regarding one or more of the predetermined muscle stimulation session and the customized muscle stimulation session, and/or receiving feedback from the user regarding one or more of the predetermined muscle stimulation session and the customized muscle stimulation session. For example, upon completion of a generated stimulation session, a user may be asked to provide feedback such as whether his/her muscle soreness has decreased and/or whether a muscle feels less fatigued. The feedback may also or instead include one or more of measured information, sensed information, information regarding an effectiveness of one or more of the predetermined muscle stimulation session and the customized muscle stimulation session, and the like. Based on the feedback, changes to parameters of a stimulation session (e.g., stimulation pulse width and stimulation time adjustments) may be automatically provided for future sessions for the classified activity (or for a similar activity). Also or instead, based on the feedback, the method 300 may include selecting one or more muscle stimulation sessions for implementation after (or before) one or more of the predetermined muscle stimulation session and the customized muscle stimulation session.

The present teachings may also or instead include techniques that either (i) create a customized stimulation session or (ii) select an existing session. For example, in one aspect, a method for customizing muscle stimulation sessions includes receiving third-party information regarding a user activity of a user, the third-party information including data received from a third-party data source; querying the user for additional, user-based information; receiving the user-based information; and creating a customized muscle stimulation session based on the third-party information and the user-based information, the customized muscle stimulation session targeting one or more muscle groups to provide blood circulation and endorphin generation stimulation modalities for flushing lactic acid from blood of the user. In another aspect, a method for selecting muscle stimulation sessions includes receiving third-party information regarding a user activity of a user, the third-party information including data received from a third-party data source; querying the user for additional, user-based information; receiving the user-based information; and reviewing a plurality of predetermined muscle stimulation sessions and providing a recommendation to the user of a predetermined muscle stimulation session from the plurality of predetermined muscle stimulation sessions, the predetermined muscle stimulation session targeting one or more muscle groups to provide blood circulation and endorphin generation stimulation modalities for flushing lactic acid from blood of the user.

It will be understood that one or more of the predetermined muscle stimulation session and the customized muscle stimulation session (or any of the stimulation sessions described herein) may include one or more of a recovery session, a drop foot assistance program, a rehabilitation program, a relaxation program, an improved performance program, and the like. It will be further understood that muscle stimulation sessions discussed herein may differ in a desired effect (e.g., treatment, fitness, performance enhancement, stimulation, and the like), application, and specific parameters (e.g., stimulation frequencies for contractions and rest periods, pulse widths, duty cycle parameters, ramp-up values, ramp-down values, burst pulse parameters, and the like). For example, a stimulation program may be targeted to do one or more of the following: improve muscular fatigue resistance (e.g., build endurance), increase muscular strength and power, improve muscle recovery (e.g., through increased blood flow), potentiate muscles, and so on. Different stimulation sessions may include different stimulation parameters.

### Real-Time Adjustments for Muscle Stimulation

The present teachings may include a capability for controlling users to adjust a stimulation session (e.g., change the session or a parameter thereof) in real-time or near real-time.

When using EMS devices that are hand-held, portable, desktop, or the like, controlling users such as doctors may perform an adjustment of stimulation parameters (e.g., stimulation pulse width, stimulation pulse frequency, total stimulation time, and the like) "on the fly," e.g., by using manual (such as dials) or digital (such as buttons) controls on the EMS devices. This "on the fly" approach may allow controlling users to accurately adjust the parameters based on a patient's real-time feedback and perceptions. However, there are limitations to the "on the fly" approach, such as: (i) it is not always possible to run and control stimulation sessions that include multiple phases following one another and where each phase has its own stimulation parameters (e.g., this may only be possible with machines having digital controls); (ii) to control more advanced stimulation parameters such as intensities, frequencies of rest intervals during contraction/rest sessions, ramp-up and -down durations, frequency/pulse width modulation parameters, inter-pulse intervals, and the like may require too many confusing buttons or dials; (iii) it may be impossible or impractical to adjust the same parameter on multiple EMS devices simultaneously; (iv) switching between patients may take time to find a correct record for a particular patient and re-adjust parameters on a machine, where some machines lack a digital memory and an ability to store and quickly recall multiple profiles; and (v) not all machines have an ability to store and lock stimulation parameters, so when a machine is rented to a patient, for example, the patient can deliberately change the parameters without a doctor knowing.

The present teachings may overcome one or more of the aforementioned challenges by using a wirelessly connected EMS device controlled from a mobile application or the like executed on a computing device such as a smartphone or tablet. That is, firmware of such EMS devices and systems (and the mobile application) can allow a doctor or other controlling user to adjust relevant stimulation parameters during an actively running stimulation session (e.g., where each phase can be adjusted separately).

Adjusted parameters can be saved (locally or to the cloud) and applied automatically for future sessions, e.g., linked to a specific user or for all future stimulation sessions of a specific stimulation program in general.

When running a session on multiple EMS devices at the same time, the same parameter (or different parameters) can be changed on multiple EMS devices as well as for multiple stimulation channels of the same EMS device. Further, some EMS devices may be adjusted while others are not.

Stimulation parameters can be also re-adjusted at a later time, e.g., before running a stimulation session or "on the fly" during a subsequently running stimulation session.

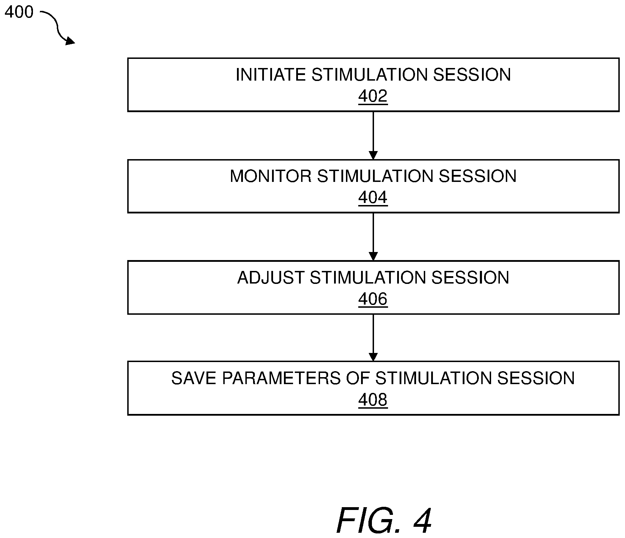

Fig. 4 is a flow chart of a method 400 for controlling a muscle stimulation session, in accordance with a representative embodiment. The method 400 may begin with a doctor or the like scheduling a new stimulation session for a patient with certain known conditions. However, it may not be known which stimulation parameters (such as pulse width, frequency of pulses, length of the contraction phase, length of the rest phase, ramp-up/down times, total number of contractions, total duration of the session, and so on) will be more comfortable/efficient for the particular patient.

As shown in step 402, the method 400 may include initiating a muscle stimulation session using a plurality of EMS devices engaged with an end user. The muscle stimulation session may be initiated by a controlling user, e.g., where the controlling user is one or more of a doctor, a trainer, and a physical therapist and the end user is a patient or client of the controlling user. For example, a doctor or the like may initiate a trial session with default parameters but then can adjust relevant parameters during the session, e.g., by asking the patient to provide feedback when parameters are adjusted, and/or by otherwise monitoring the patient using sensors or the like (or mere observation).

As shown in step 404, the method 400 may include monitoring the muscle stimulation session including analyzing one or more attributes of the end user and one or more parameters of the plurality of EMS devices. This may include using sensors (e.g., physiological sensors), and/or through querying the patient or otherwise receiving feedback from the patient.

The one or more attributes of the end user that are analyzed in step 404 may include a sensed or measured physiological parameter of the end user (e.g., heart rate, blood pressure, pulse oximetry, respiratory rate and information, muscle contractions, mechanomyography (MMG) readings, temperature, and the like). The one or more attributes of the end user that are analyzed in step 404 may also or instead include feedback received from querying the end user. In this manner, the method 400 may further include querying the end user for feedback related to the muscle stimulation session. Therefore, as described above, monitoring the muscle stimulation session may include analyzing a combination of sensor data and feedback received from querying the end user.

The one or more parameters of the plurality of EMS devices that are analyzed in step 404 may include at least one of a pulse width, a frequency of pulses, a length of a contraction phase, a length of a rest phase, a rest period, a ramp-up time, a ramp-down time, duty cycle parameters, a number of contractions, a duration, burst pulse parameters, waveform shape, interphase intervals, and the like.

As shown in step 406, the method 400 may include adjusting the stimulation session, e.g., during the stimulation session based on the monitoring and feedback. Thus, the method 400 may include adjusting the muscle stimulation session based on the analysis of the aforementioned one or more attributes of the end user and the aforementioned one or more parameters of the plurality of EMS devices. Further, the stimulation session may be continuously monitored and adjusted by a controlling user.

Adjusting the stimulation session may include an adjustment to one or more parameters of the plurality of EMS devices, e.g., the same parameters of the plurality of EMS devices that are analyzed in step 404. In this manner, adjusting the muscle stimulation session may include altering one or more of a pulse width, a frequency of pulses, a length of a contraction phase, a length of a rest phase, a ramp-up time, a ramp-down time, a number of contractions, a duration of the muscle stimulation session, and the like. Adjusting the stimulation session may also or instead include altering a location of one or more of the plurality of EMS devices, including adding or removing devices.

As shown in step 408, the method 400 may include saving parameters for a stimulation session, and/or saving feedback or data obtained from the muscle stimulation session. In this manner, the method 400 may include saving parameters of the muscle stimulation session after adjusting the muscle stimulation session. For example, a doctor may save the parameters for a particular patient, and the next time the doctor runs the same scheduled session for the patient, the parameters may be automatically preloaded (e.g., where they override the default parameters of a selected session). In this manner, the method 400 can also include overriding default parameters with the saved parameters. Of course, the doctor can further re-adjust parameters either before or during a stimulation session. To that end, the muscle stimulation session that is initiated may include a trial session with default parameters, where the default parameters are adjustable.

The above systems, devices, methods, processes, and the like may be realized in hardware, software, or any combination of these suitable for a particular application. The hardware may include a general-purpose computer and/or dedicated computing device. This includes realization in one or more microprocessors, microcontrollers, embedded microcontrollers, programmable digital signal processors or other programmable devices or processing circuitry, along with internal and/or external memory. This may also, or instead, include one or more application specific integrated circuits, programmable gate arrays, programmable array logic components, or any other device or devices that may be configured to process electronic signals. It will further be appreciated that a realization of the processes or devices described above may include computer-executable code created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software. In another aspect, the methods may be embodied in systems that perform the steps thereof, and may be distributed across devices in a number of ways. At the same time, processing may be distributed across devices such as the various systems described above, or all of the functionality may be integrated into a dedicated, standalone device or other hardware. In another aspect, means for performing the steps associated with the processes described above may include any of the hardware and/or software described above. All such permutations and combinations are intended to fall within the scope of the present disclosure.

Embodiments disclosed herein may include computer program products comprising computer-executable code or computer-usable code that, when executing on one or more computing devices, performs any and/or all of the steps thereof. The code may be stored in a non-transitory fashion in a computer memory, which may be a memory from which the program executes (such as random-access memory associated with a processor), or a storage device such as a disk drive, flash memory or any other optical, electromagnetic, magnetic, infrared, or other device or combination of devices. In another aspect, any of the systems and methods described above may be embodied in any suitable transmission or propagation medium carrying computer-executable code and/or any inputs or outputs from same.

The foregoing description, for purpose of explanation, has been described with reference to specific embodiments. However, the illustrative discussions above are not intended to be exhaustive or to limit the disclosure to the precise forms disclosed. Many modifications and variations are possible in view of the above teachings.

Unless the context clearly requires otherwise, throughout the description, the words "comprise," "comprising," "include," "including," and the like are to be construed in an inclusive sense as opposed to an exclusive or exhaustive sense; that is to say, in a sense of "including, but not limited to." Additionally, the words "herein," "hereunder," "above," "below," and words of similar import refer to this application as a whole and not to any particular portions of this application.

It will be appreciated that the devices, systems, and methods described above are set forth by way of example and not of limitation. For example, regarding the methods provided above, absent an explicit indication to the contrary, the disclosed steps may be modified, supplemented, omitted, and/or re-ordered without departing from the scope of this disclosure. Numerous variations, additions, omissions, and other modifications will be apparent to one of ordinary skill in the art. In addition, the order or presentation of method steps in the description and drawings above is not intended to require this order of performing the recited steps unless a particular order is expressly required or otherwise clear from the context.

The method steps of the implementations described herein are intended to include any suitable method of causing such method steps to be performed, consistent with the patentability of the following claims, unless a different meaning is expressly provided or otherwise clear from the context. So, for example performing the step of X includes any suitable method for causing another party such as a remote user, a remote processing resource (e.g., a server or cloud computer) or a machine to perform the step of X. Similarly, performing steps X, Y and Z may include any method of directing or controlling any combination of such other individuals or resources to perform steps X, Y and Z to obtain the benefit of such steps. Thus, method steps of the implementations described herein are intended to include any suitable method of causing one or more other parties or entities to perform the steps, consistent with the patentability of the following claims, unless a different meaning is expressly provided or otherwise clear from the context. Such parties or entities need not be under the direction or control of any other party or entity, and need not be located within a particular jurisdiction.

It will be appreciated that the methods and systems described above are set forth by way of example and not of limitation. Numerous variations, additions, omissions, and other modifications will be apparent to one of ordinary skill in the art. In addition, the order or presentation of method steps in the description and drawings above is not intended to require this order of performing the recited steps unless a particular order is expressly required or otherwise clear from the context. Thus, while particular embodiments have been shown and described, it will be apparent to those skilled in the art that various changes and modifications in form and details may be made therein without departing from the scope of this disclosure and are intended to form a part of the invention as defined by the following claims

## Claims

1. A system (100) for muscle stimulation, the system comprising:
a first computing device (112) associated with a controlling user (110);
a first set of electrical muscle stimulation (EMS) devices (131) configured to be coupled to a first end user (121) of the system, wherein one or more EMS devices in the first set of EMS devices is in communication with a first processor (132a) and a first memory (134a), and wherein one or more of the first processor and the first memory is configured to receive and store instructions from the first computing device;
a second set of EMS devices (133) configured to be coupled to a second end user (123) of the system, wherein one or more EMS devices in the second set of EMS devices is in communication with a second processor (132b) and a second memory (134b), and wherein one or more of the second processor and the second memory is configured to receive and store instructions from the first computing device; and
one or more communications channels (141) between the first computing device and (i) one or more of the first processor and the first memory and (ii) one or more of the second processor and the second memory, the one or more communications channels configured to transmit data containing one or more stimulation programs from the first computing device to facilitate operation of the first set of EMS devices and the second set of EMS devices simultaneously, and the one or more communications channels switchable to stop communication from the first computing device after a predetermined transmission of a stimulation program for independent implementation of the stimulation program by an EMS device;
wherein the first end user and the second end user are in different geographic locations.

2. The system of claim 1, wherein the first computing device (112) is configured to allow the controlling user (110) to adjust stimulation parameters of the first and second sets of EMS devices (131, 133) during an actively running stimulation session.

3. The system of claim 1, wherein the one or more communications channels (141) are configured to transmit the data to (i) one or more of the first processor (132a) and the first memory (134a) and (ii) one or more of the second processor (132b) and the second memory (134b) for implementation of the one or more stimulation programs on one or more of the EMS devices in the first set of EMS devices (131) and one or more of the EMS devices in the second set of EMS devices (133).

4. The system of claim 2, wherein, after transmission of the data, the one or more communications channels (141) are configured to stop transmissions between the first computing device (112) and one or more of the first processor (132a), the first memory (134a), the second processor (132b), and the second memory (134b).

5. The system of claim 1, wherein one or more of the first processor (132a) and the first memory (134a) is integral with one or more of the EMS devices in the first set of EMS devices (131).

6. The system of claim 1, wherein one or more of the second processor (132b) and the second memory (134b) is integral with one or more of the EMS devices in the second set of EMS devices (133).

7. The system of claim 1, further comprising a second computing device (122) associated with the first end user (121) of the system, wherein one or more of the first processor (132a) and the first memory (134a) is disposed on the second computing device.

8. The system of claim 7, wherein the second computing device (122) is separate and distinct from, but in communication with, the first set of EMS devices (131); and, optionally, wherein the second computing device (122) is one or more of a smartphone and a tablet.

9. The system of claim 1, further comprising a third computing device (124) associated with the second end user (123) of the system, wherein one or more of the second processor (132b) and the second memory (134b) is disposed on the third computing device.

10. The system of claim 9, wherein the third computing device (124) is separate and distinct from, but in communication with, the second set of EMS devices (133).

11. The system of claim 10, wherein the third computing device (124) is one or more of a smartphone and a tablet.

12. The system of claim 1, wherein one or more of the first memory (134a) and the second memory (134b) is configured to store the stimulation program.

13. The system of claim 1, wherein one or more of the first memory (134a) and the second memory (134b) is configured to store historical data related to EMS devices and stimulation sessions.

14. The system of claim 1, wherein the controlling user (110) is one or more of a doctor and a physical therapist; and, optionally, wherein the first end user (121) and the second end user (123) are patients of the controlling user (110).

15. The system of claim 1, wherein the first computing device (112) is one or more of a smartphone and a tablet.

## Patentansprüche

1. System (100) zur Muskelstimulation, wobei das System Folgendes umfasst:
eine erste Computervorrichtung (112), die einem steuernden Benutzer (110) zugeordnet ist;
einen ersten Satz von Vorrichtungen zur elektrischen Muskelstimulation (EMS-Vorrichtungen) (131), die konfiguriert sind, um mit einem ersten Endbenutzer (121) des Systems gekoppelt zu werden, wobei eine oder mehrere EMS-Vorrichtungen in dem ersten Satz von EMS-Vorrichtungen mit einem ersten Prozessor (132a) und einem ersten Speicher (134a) in Kommunikation stehen, und wobei einer oder mehrere von dem ersten Prozessor und dem ersten Speicher konfiguriert sind, um Anweisungen von der ersten Computervorrichtung zu empfangen und zu speichern;
einen zweiten Satz von EMS-Vorrichtungen (133), die konfiguriert sind, um mit einem zweiten Endbenutzer (123) des Systems gekoppelt zu werden, wobei eine oder mehrere EMS-Vorrichtungen in dem zweiten Satz von EMS-Vorrichtungen mit einem zweiten Prozessor (132b) und einem zweiten Speicher (134b) in Kommunikation stehen, und wobei einer oder mehrere von dem zweiten Prozessor und dem zweiten Speicher konfiguriert sind, um Anweisungen von der ersten Computervorrichtung zu empfangen und zu speichern; und
einen oder mehrere Kommunikationskanäle (141) zwischen der ersten Computervorrichtung und (i) einem oder mehreren von dem ersten Prozessor und dem ersten Speicher und (ii) einem oder mehreren von dem zweiten Prozessor und dem zweiten Speicher, wobei der eine oder die mehreren Kommunikationskanäle konfiguriert sind, um Daten, die ein oder mehrere Stimulationsprogramme enthalten, von der ersten Computervorrichtung zu übertragen, um gleichzeitig den Betrieb des ersten Satzes von EMS-Vorrichtungen und des zweiten Satzes von EMS-Vorrichtungen zu erleichtern, und der eine oder die mehreren Kommunikationskanäle umschaltbar sind, um die Kommunikation von der ersten Computervorrichtung nach einer vorbestimmten Übertragung eines Stimulationsprogramms für eine unabhängige Implementierung des Stimulationsprogramms durch eine EMS-Vorrichtung zu stoppen;
wobei sich der erste Endbenutzer und der zweite Endbenutzer an unterschiedlichen geografischen Standorten befinden.

2. System nach Anspruch 1, wobei die erste Computervorrichtung (112) konfiguriert ist, um es dem steuernden Benutzer (110) zu erlauben, Stimulationsparameter des ersten und zweiten Satzes von EMS-Vorrichtungen (131, 133) während einer aktiv laufenden Stimulationssitzung einzustellen.

3. System nach Anspruch 1, wobei der eine oder die mehreren Kommunikationskanäle (141) konfiguriert sind, um die Daten an (i) einen oder mehrere von dem ersten Prozessor (132a) und dem ersten Speicher (134a) und (ii) einen oder mehrere von dem zweiten Prozessor (132b) und dem zweiten Speicher (134b) zur Implementierung des einen oder der mehreren Stimulationsprogramme auf einer oder mehreren der EMS-Vorrichtungen in dem ersten Satz von EMS-Vorrichtungen (131) und einer oder mehreren der EMS-Vorrichtungen in dem zweiten Satz von EMS-Vorrichtungen (133) zu übertragen.

4. System nach Anspruch 2, wobei nach der Übertragung der Daten der eine oder die mehreren Kommunikationskanäle (141) konfiguriert sind, um Übertragungen zwischen der ersten Computervorrichtung (112) und einem oder mehreren von dem ersten Prozessor (132a), dem ersten Speicher (134a), dem zweiten Prozessor (132b) und dem zweiten Speicher (134b) zu stoppen.

5. System nach Anspruch 1, wobei einer oder mehrere von dem ersten Prozessor (132a) und dem ersten Speicher (134a) einstückig mit einer oder mehreren der EMS-Vorrichtungen in dem ersten Satz von EMS-Vorrichtungen (131) ausgebildet sind.

6. System nach Anspruch 1, wobei einer oder mehrere von dem zweiten Prozessor (132b) und dem zweiten Speicher (134b) einstückig mit einer oder mehreren der EMS-Vorrichtungen in dem zweiten Satz von EMS-Vorrichtungen (133) ausgebildet sind.

7. System nach Anspruch 1, das ferner eine zweite Computervorrichtung (122) umfasst, die dem ersten Endbenutzer (121) des Systems zugeordnet ist, wobei einer oder mehrere von dem ersten Prozessor (132a) und dem ersten Speicher (134a) auf der zweiten Computervorrichtung angeordnet sind.

8. System nach Anspruch 7, wobei die zweite Computervorrichtung (122) separat und getrennt von, aber in Kommunikation mit dem ersten Satz von EMS-Vorrichtungen (131) ist; und wobei die zweite Computervorrichtung (122) optional eines oder mehrere von einem Smartphone und einem Tablet ist.

9. System nach Anspruch 1, das ferner eine dritte Computervorrichtung (124) umfasst, die dem zweiten Endbenutzer (123) des Systems zugeordnet ist, wobei einer oder mehrere von dem zweiten Prozessor (132b) und dem zweiten Speicher (134b) auf der dritten Computervorrichtung angeordnet sind.

10. System nach Anspruch 9, wobei die dritte Computervorrichtung (124) separat und getrennt von, aber in Kommunikation mit dem zweiten Satz von EMS-Vorrichtungen (133) ist.

11. System nach Anspruch 10, wobei die dritte Computervorrichtung (124) eines oder mehrere von einem Smartphone und einem Tablet ist.

12. System nach Anspruch 1, wobei einer oder mehrere von dem ersten Speicher (134a) und dem zweiten Speicher (134b) konfiguriert sind, um das Stimulationsprogramm zu speichern.

13. System nach Anspruch 1, wobei einer oder von dem ersten Speicher (134a) und dem zweiten Speicher (134b) konfiguriert sind, um Verlaufsdaten in Bezug auf EMS-Vorrichtungen und Stimulationssitzungen zu speichern.

14. System nach Anspruch 1, wobei der steuernde Benutzer (110) einer oder mehrere von einem Arzt und einem Physiotherapeuten ist; und wobei der erste Endbenutzer (121) und der zweite Endbenutzer (123) optional Patienten des steuernden Benutzers (110) sind.

15. System nach Anspruch 1, wobei die dritte Computervorrichtung (112) eines oder mehrere von einem Smartphone und einem Tablet ist.

## Revendications

1. Système (100) de stimulation musculaire, le système comprenant :
un premier dispositif informatique (112) associé à un utilisateur régulateur (110),
un premier ensemble de dispositifs de stimulation musculaire électrique (dispositifs EMS) (131) configurés pour être couplés à un premier utilisateur final (121) du système, un ou plusieurs dispositifs EMS du premier ensemble de dispositifs EMS étant en communication avec un premier processeur (132a) et une première mémoire (134a), et un ou plusieurs du premier processeur et de la première mémoire étant configurés pour recevoir et stocker des instructions provenant du premier dispositif informatique,
un deuxième ensemble de dispositifs EMS (133) configurés pour être couplés à un deuxième utilisateur final (123) du système, un ou plusieurs dispositifs EMS du deuxième ensemble de dispositifs EMS étant en communication avec un deuxième processeur (132b) et une deuxième mémoire (134b), et un ou plusieurs du deuxième processeur et de la deuxième mémoire étant configurés pour recevoir et stocker des instructions provenant du premier dispositif informatique, et
un ou plusieurs canaux de communication (141) entre le premier dispositif informatique et (i) un ou plusieurs du premier processeur et de la première mémoire et (ii) un ou plusieurs du deuxième processeur et de la deuxième mémoire, les un ou plusieurs canaux de communication étant configurés pour transmettre des données contenant un ou plusieurs programmes de stimulation à partir du premier dispositif informatique pour faciliter le fonctionnement du premier ensemble de dispositifs EMS et du deuxième ensemble de dispositifs EMS simultanément, et les un ou plusieurs canaux de communication pouvant être l'objet d'une commutation pour arrêter la communication à partir du premier dispositif informatique après une transmission prédéterminée d'un programme de stimulation pour une mise en œuvre indépendante du programme de stimulation par un dispositif EMS ;
ledit premier utilisateur final et ledit deuxième utilisateur final se trouvant en des emplacements géographiques différents.

2. Système selon la revendication 1, dans lequel le premier dispositif informatique (112) est configuré pour permettre à l'utilisateur régulateur (110) d'ajuster des paramètres de stimulation des premier et deuxième ensembles de dispositifs EMS (131, 133) pendant une session de stimulation en cours d'exécution.

3. Système selon la revendication 1, dans lequel les un ou plusieurs canaux de communication (141) sont configurés pour transmettre les données à (i) un ou plusieurs du premier processeur (132a) et de la première mémoire (134a) et (ii) un ou plusieurs du deuxième processeur (132b) et de la deuxième mémoire (134b) pour la mise en œuvre des un ou plusieurs programmes de stimulation sur un ou plusieurs des dispositifs EMS du premier ensemble de dispositifs EMS (131) et un ou plusieurs des dispositifs EMS du deuxième ensemble de dispositifs EMS (133).

4. Système selon la revendication 2, dans lequel, après la transmission des données, les un ou plusieurs canaux de communication (141) sont configurés pour arrêter les transmissions entre le premier dispositif informatique (112) et un ou plusieurs éléments parmi : le premier processeur (132a), la première mémoire (134a), le deuxième processeur (132b) et la deuxième mémoire (134b).

5. Système selon la revendication 1, dans lequel un ou plusieurs du premier processeur (132a) et de la première mémoire (134a) font partie intégrante d'un ou de plusieurs des dispositifs EMS du premier ensemble de dispositifs EMS (131).

6. Système selon la revendication 1, dans lequel un ou plusieurs de deuxième processeur (132b) et de la deuxième mémoire (134b) font partie intégrante d'un ou de plusieurs des dispositifs EMS du deuxième ensemble de dispositifs EMS (133).

7. Système selon la revendication 1, comprenant en outre un deuxième dispositif informatique (122) associé au premier utilisateur final (121) du système, un ou plusieurs du premier processeur (132a) et de la première mémoire (134a) étant disposés sur le deuxième dispositif informatique.

8. Système selon la revendication 7, dans lequel le deuxième dispositif informatique (122) est séparé et distinct du premier ensemble de dispositifs EMS (131), mais en communication avec celui-ci ; et éventuellement dans lequel le deuxième dispositif informatique (122) est un ou plusieurs parmi un smartphone et une tablette.

9. Système selon la revendication 1, comprenant en outre un troisième dispositif informatique (124) associé au deuxième utilisateur final (123) du système, un ou plusieurs du deuxième processeur (132b) et de la deuxième mémoire (134b) étant disposés sur le troisième dispositif informatique.

10. Système selon la revendication 9, dans lequel le troisième dispositif informatique (124) est séparé et distinct du deuxième ensemble de dispositifs EMS (133), mais en communication avec celui-ci.

11. Système selon la revendication 10, dans lequel le troisième dispositif informatique (124) est un ou plusieurs parmi un smartphone et une tablette.

12. Système selon la revendication 1, dans lequel une ou plusieurs de la première mémoire (134a) et de la deuxième mémoire (134b) sont configurées pour stocker le programme de stimulation.

13. Système selon la revendication 1, dans lequel une ou plusieurs de la première mémoire (134a) et de la deuxième mémoire (134b) sont configurées pour stocker des données historiques liées aux dispositifs EMS et aux sessions de stimulation.

14. Système selon la revendication 1, dans lequel l'utilisateur régulateur (110) est un ou plusieurs d'un médecin et d'un physiothérapeute ; et éventuellement dans lequel le premier utilisateur final (121) et le deuxième utilisateur final (123) sont des patients de l'utilisateur régulateur (110).

15. Système selon la revendication 1, dans lequel le premier dispositif informatique (112) est un ou plusieurs parmi un smartphone et une tablette.
